# EUROPEAN PATENT APPLICATION

(11) **EP 4 227 407 A1**
(43) Date of publication of application: **16.08.2023**
(21) Application number: 21877683.9
(22) Date of filing: 06.10.2021
(51) Int. Cl.: C12N 5/10, C07K 16/10, C12N 5/0784, C12N 5/0786, C12N 7/02, C12N 15/19, C12N 15/57, C12Q 1/6851, C12Q 1/686, C12Q 1/70, G01N 33/15, G01N 33/50, G01N 33/53, G01N 33/68

(54) **CORONAVIRUS-INFECTIVE CELLS AND METHOD OF PREPARING SAME**

(30) Priority: 07.10.2020 JP 2020170141
(71) Applicant: Mican Technologies Inc., Kyoto-shi, Kyoto 615-8245 (JP); OSAKA UNIVERSITY, Suita-shi Osaka 565-0871 (JP)
(72) Inventor: SHIMIZU, Jun, Kyoto-shi, Kyoto 615-8245 (JP); TANGA, Naomi, Kyoto-shi, Kyoto 615-8245 (JP); YAMADA, Misuzu, Kyoto-shi, Kyoto 615-8245 (JP); MIYAZAKI, Kazuo, Kyoto-shi, Kyoto 615-8245 (JP); SHIODA, Tatsuo, Suita-shi, Osaka 565-0871 (JP); NAKAYAMA, Emi, Suita-shi, Osaka 565-0871 (JP); SASAKI, Tadahiro, Suita-shi, Osaka 565-0871 (JP); KOKETSU, Ritsuko, Suita-shi, Osaka 565-0871 (JP)
(74) Representative: J A Kemp LLP
(86) International application number: PCT/JP2021/037069
(87) International publication number: WO 2022/075384

(57) **Abstract**

The present invention provides human cells that can be stably infected with coronavirus, as human cells for research on infection with coronavirus such as SARS-CoV-2, as well as, by utilizing the cells, a method for confirming the presence of a coronavirus in a sample, a method for producing a coronavirus, and a method for evaluating the presence or absence of anti-coronavirus activity of a test sample or a substance having an anti-coronavirus neutralizing action in a test sample. Specifically, the present invention relates to a method for preparing a coronavirus-infectious immortalized human myeloid cell, including introducing ACE2 and TMPRSS2 into an immortalized human myeloid cell, and the like.

## Description

### [Technical Field]

The present invention relates to cells to evaluate coronavirus infection, a preparation method thereof, a method for evaluating coronavirus infection by using the cells, and the like.

### [Background Art]

At present, therapeutic drugs and vaccines are being developed against the COVID-19 infectious syndrome, which is raging all over the world. In such development, it is necessary to achieve continuous proliferation of SARS-CoV-2, which is the causative virus of COVID-19 infectious syndrome. For this purpose, it is necessary to infect host cells with SARS-CoV-2.

At present, it has been reported that SARS-CoV-2 infects and proliferates in various cells derived from the intestinal tract, bile duct, and liver, including VeroE6 cells derived from African green monkey kidney (Non Patent Literatures 1, 2, and 3). However, a SARS-CoV-2 infection model in human blood-lineage immune cell, which is the source of cytokine production, has not been reported.

### [Citation List]

### [Non Patent Literature]

[NPL 1]
   PNAS, 2020 Mar, 117(13) 7001-7003
[NPL 2]
   Cell STEM CELL, 2020 July 27, 125-136
[NPL 3]
   Science, 2020, 369, 50-54

### [Summary of Invention]

### [Technical Problem]

The present invention has been made in view of the above-mentioned problems of the prior art, and aims to provide human cells that can be stably infected with coronavirus, as human cells for research on infection with coronavirus such as SARS-CoV-2.

The present invention also aims to provide a method for confirming the presence of a coronavirus in a sample, a method for producing a coronavirus, and a method for evaluating the presence or absence of anti-coronavirus activity of a test sample or a substance having an anti-coronavirus neutralizing action in a test sample.

### [Solution to Problem]

The present inventors have studied cells suitable for infection with SARS-CoV-2 and succeeded in preparing cells in which a coronavirus efficiently infects and proliferates, by introducing ACE2 and TMPRSS2 into proliferative immortalized human monocytes derived from iPS cells or the like. Also, coronavirus infection of the prepared cells caused the production of inflammatory cytokines. Furthermore, the present inventors have found that differentiation of the obtained cells increases virus proliferation and cytokine release associated with coronavirus infection. Therefore, they have found that use of these cells makes it possible to confirm the presence of the virus by using an increase in the virus level and/or the amount of inflammatory cytokine production as an index.

Specifically, the present invention relates to the following invention.
(1) A method for preparing a coronavirus-infectious immortalized human myeloid cell, comprising introducing ACE2 and TMPRSS2 into an immortalized human myeloid cell.
(2) The preparation method of (1), wherein the aforementioned immortalized human myeloid cell is a cell that expresses M-CSF and/or GM-CSF.
(3) The preparation method of (1), comprising introducing M-CSF and/or GM-CSF into the aforementioned immortalized myeloid cell, and
   introducing ACE2 and TMPRSS2 into the obtained immortalized myeloid cell that expresses M-CSF and/or GM-CSF.
(4) The preparation method of any one of (1) to (3), wherein the aforementioned ACE2 and the aforementioned TMPRSS2 are introduced by lentivirus.
(5) The preparation method of any one of (1) to (4), wherein the aforementioned immortalized human myeloid cell is an immortalized human monocyte.
(6) A method for preparing a dendritic cell that expresses ACE2 and TMPRSS2, comprising differentiating the human immortalized monocyte obtained by the method of (5) into a dendritic cell.
(7) The preparation method of any one of (1) to (6), wherein the aforementioned coronavirus is SARS-CoV-2.
(8) An immortalized human myeloid cell that expresses ACE2 and TMPRSS2.
(9) The immortalized human myeloid cell of (8) that further expresses M-CSF and/or GM-CSF.
(10) The cell of (8) or (9), wherein the aforementioned immortalized human myeloid cell is an immortalized human monocyte.
(11) The cell of (8) or (9), wherein the aforementioned immortalized human myeloid cell is a human dendritic cell.
(12) A method for determining the presence of a coronavirus in a sample, comprising adding a sample to a culture solution of the immortalized human myeloid cell of any one of (9) to (11), and
   detecting a coronavirus or measuring the level of a coronavirus in a culture supernatant of the aforementioned cell.
(13) The method of (12), wherein the aforementioned detection of coronavirus or the aforementioned measurement of the level of coronavirus in the culture supernatant is performed 1 to 7 days after the addition of the sample.
(14) The method of (12) or (13), wherein the aforementioned detection of coronavirus or measurement of the level of coronavirus is performed by detection of RNA or measurement of RNA level of the aforementioned coronavirus.
(15) The method of (14), wherein the aforementioned detection of RNA or measurement of RNA level of coronavirus is performed by quantitative PCR.
(16) The method of (12) or (13), wherein the aforementioned detection of coronavirus or measurement of the level of coronavirus is performed by detection of an inflammatory cytokine or measurement of the level of inflammatory cytokine in the aforementioned culture supernatant.
(17) The method of (16), wherein the aforementioned inflammatory cytokine is IL-6.
(18) A method for producing a coronavirus, comprising adding a coronavirus to a culture solution of the immortalized human myeloid cell of any one of (9) to (11) to infect the cell, and
   recovering the coronavirus in the aforementioned culture solution of the aforementioned cell.
(19) The method of (18), wherein the coronavirus in the aforementioned culture solution is recovered 1 to 7 days after the addition of the coronavirus.
(20) A method for evaluating an anti-coronavirus activity of a test sample, comprising adding a coronavirus to a culture solution of the immortalized human myeloid cell of any one of (9) to (11) in the presence of the aforementioned test sample,
   measuring the level of the aforementioned coronavirus in a culture supernatant of the aforementioned cell, and
   evaluating the test sample as having an anti-coronavirus activity when the level of the coronavirus in the culture supernatant to which the coronavirus has been added in the presence of the test sample is lower than the level of the coronavirus in the culture supernatant to which the coronavirus has been added in the absence of the test sample.
(21) A method for identifying whether a substance having a coronavirus neutralization action is contained in a test sample, comprising mixing a coronavirus with a culture solution of the immortalized human myeloid cell of any one of (9) to (11) in the presence of the aforementioned test sample,
   measuring the level of the aforementioned coronavirus in a culture supernatant of the aforementioned cell, and
   evaluating the test sample as containing a substance having a coronavirus neutralization action when the level of the coronavirus in the culture supernatant to which the coronavirus has been added in the presence of the test sample is lower than the level of the coronavirus in the culture supernatant to which the coronavirus has been added in the absence of the test sample.
(22) The method of (21), wherein the aforementioned test sample is a blood sample.
(23) The method of (21) or (22), wherein the aforementioned substance having a coronavirus neutralization action is a coronavirus neutralizing antibody.
(24) A method for evaluating a neutralizing antibody inducibility of a coronavirus vaccine, comprising performing the method of (22) or (23) by using a blood sample derived from a mammal inoculated with the vaccine as a test sample.
(25) The method of any one of (20) to (24), wherein the measurement of the level of the aforementioned coronavirus in the culture supernatant is performed 1 to 7 days after the addition of the test sample or the sample.
(26) The method of any one of (20) to (25), wherein the aforementioned detection of coronavirus or measurement of the level of coronavirus is performed by detection of RNA or measurement of RNA level of the aforementioned coronavirus.
(27) The method of (26), wherein the aforementioned measurement of the RNA level of coronavirus is performed by quantitative PCR.
(28) The method of any one of (20) to (25), wherein the aforementioned measurement of the level of coronavirus is performed by measurement of the level of an inflammatory cytokine in the aforementioned culture supernatant.
(29) The method of (28), wherein the aforementioned inflammatory cytokine is IL-6.

### [Advantageous Effects of Invention]

Coronavirus infectious human cells can be stably supplied by using the cell of the present invention. As a result, coronavirus infection can be studied using the cells of the human immune system. In addition, cells having coronavirus infectivity can be provided stably. By using the coronavirus-infectious immortalized human myeloid cells of the present invention, it becomes possible to infect human immune system cells with coronaviruses such as SARS-CoV-2 and the like, and coronavirus infection can be evaluated under conditions closer to those in human vivo. By using the coronavirus-infectious cells of the present invention, moreover, viral infection experiments in vitro can be performed. This makes it possible to elucidate the mechanism by which coronaviruses infect human immune system cells, and the mechanism that causes exacerbation and cytokine storm involving immune cells. Furthermore, the coronavirus-infectious cells provided by the present invention can be used for the development of therapeutic drugs and vaccines.

### [Brief Description of Drawings]

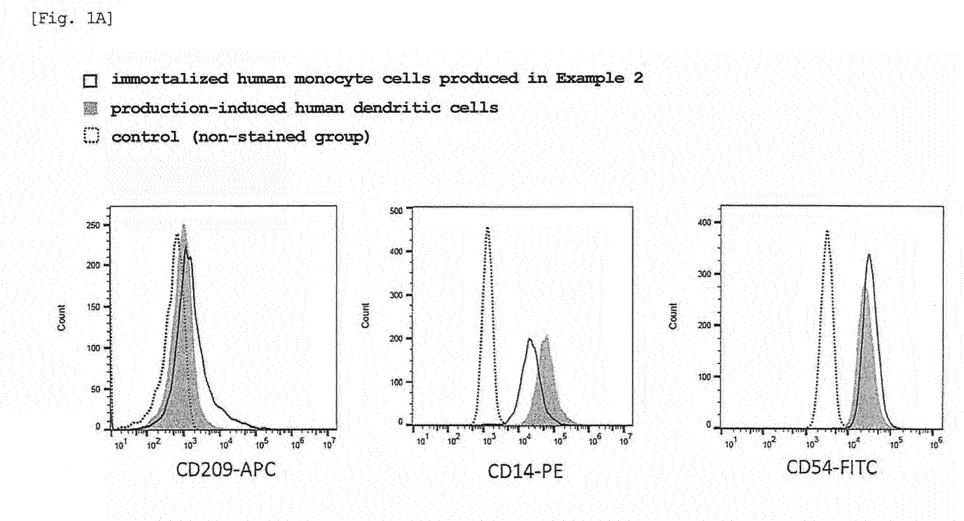

Fig. 1A shows values of changes, as measured using a flow cytometer, in CD14, CD54, and CD209 as characteristic cell surface markers in produced immortalized human myeloid cells (K-ML2) and dendritic cells induced and produced by IL-4.

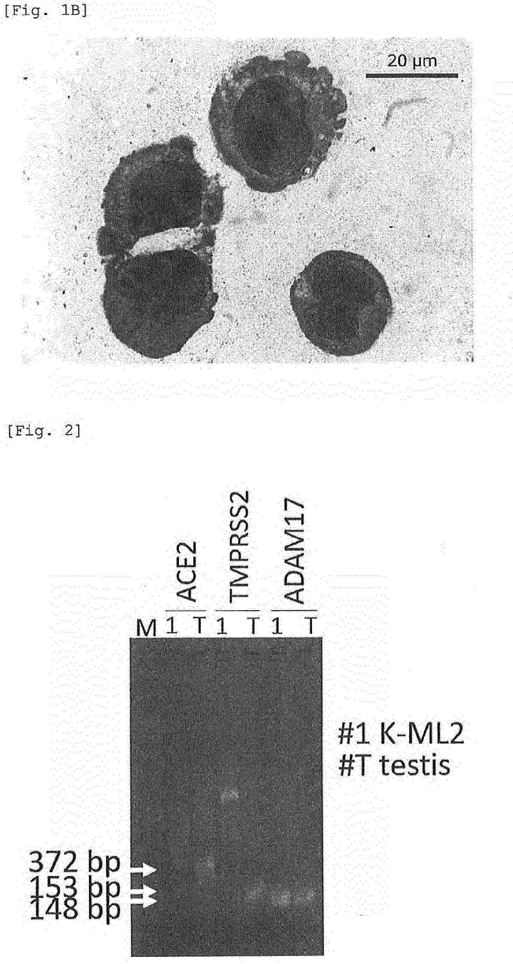

Fig. 1B is a Giemsa-stained diagram of the produced immortalized human myeloid cells (K-ML2).
Fig. 2 is an electrophoresis photograph of transcripts obtained by quantitative PCR to confirm the expression levels of various genes (ACE2, TMPRSS2, ADAM17) in the prepared immortalized human myeloid cells (K-ML2).

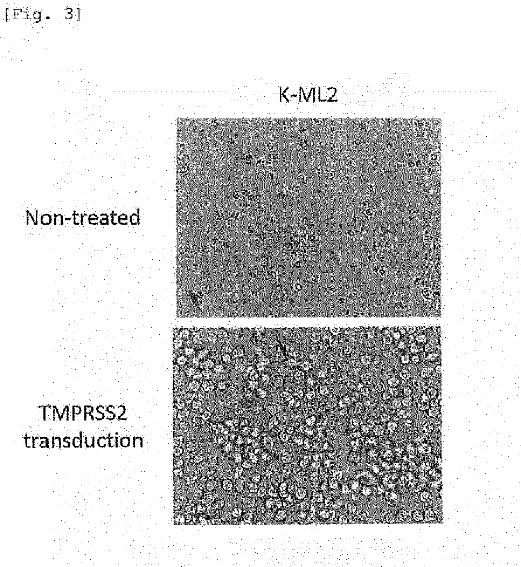

Fig. 3 shows photographs of cells of cultured K-ML2 transfected with a vector containing the TMPRSS2 gene and a drug resistance gene (TMPRSS2 transduction; lower photograph) and non-transfected K-ML2 (Non-treated; upper photograph) in the presence of drug.

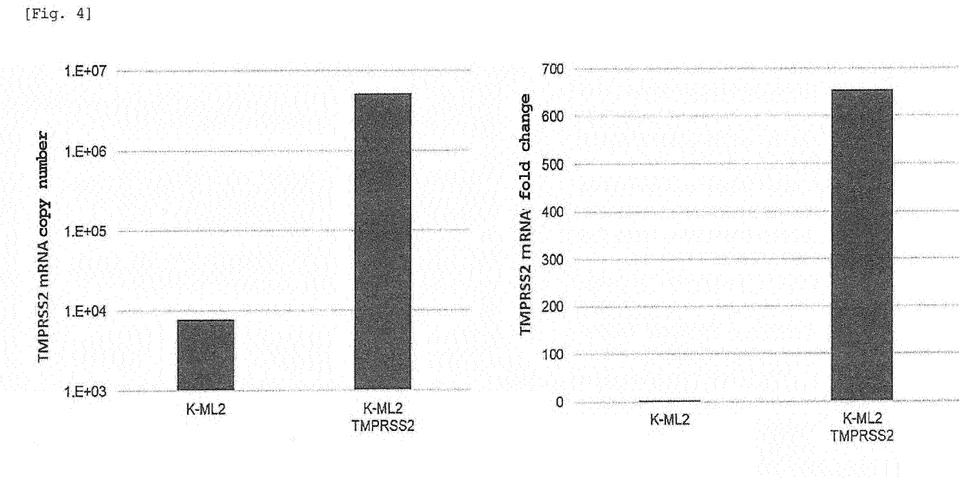

Fig. 4 shows graphs with the measurement results by quantitative PCR of the amount of TMPRSS2 RNA in K-ML2 and K-ML2 with the TMPRSS2 gene introduced thereinto (K-ML2/TMPRSS2). The left figure shows the copy number per sample, and the right figure shows the numerical value (fold change) obtained by dividing the copy number by the copy number measured with K-ML2. The horizontal axis of the graph indicates the cell name, and the vertical axis of the graph indicates the copy number of TMPRSS2 RNA (left figure) and the fold change (right figure).

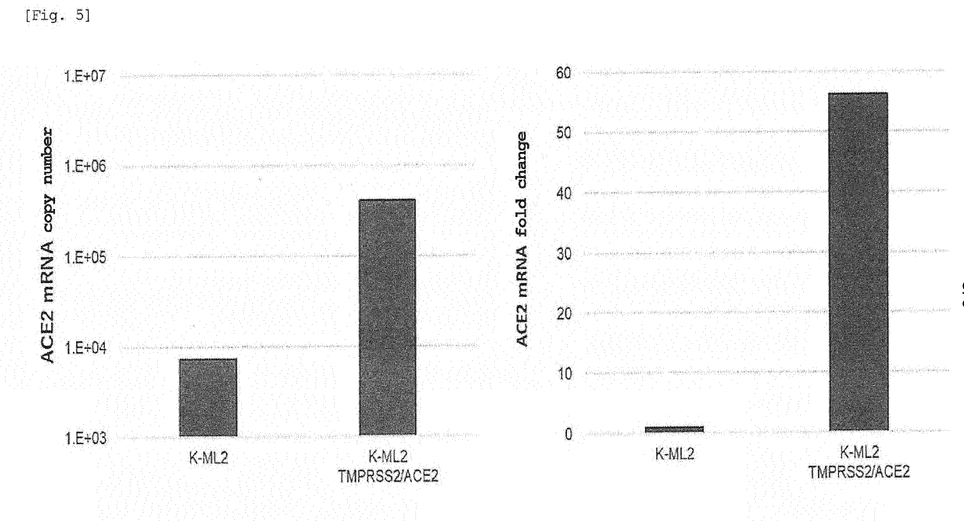

Fig. 5 shows graphs with the measurement results by quantitative PCR of the amount of ACE2 RNA in immortalized human myeloid cells with the ACE2 and TMPRSS2 genes introduced thereinto (K-ML2/TMPRSS2/ACE2). The left figure shows the copy number per sample, and the right figure shows the numerical value (fold change) obtained by dividing the copy number by the copy number measured with K-ML2. The horizontal axis of the graph indicates the cell name, and the vertical axis of the graph indicates the copy number of ACE2 RNA (left figure) and the fold change (right figure).

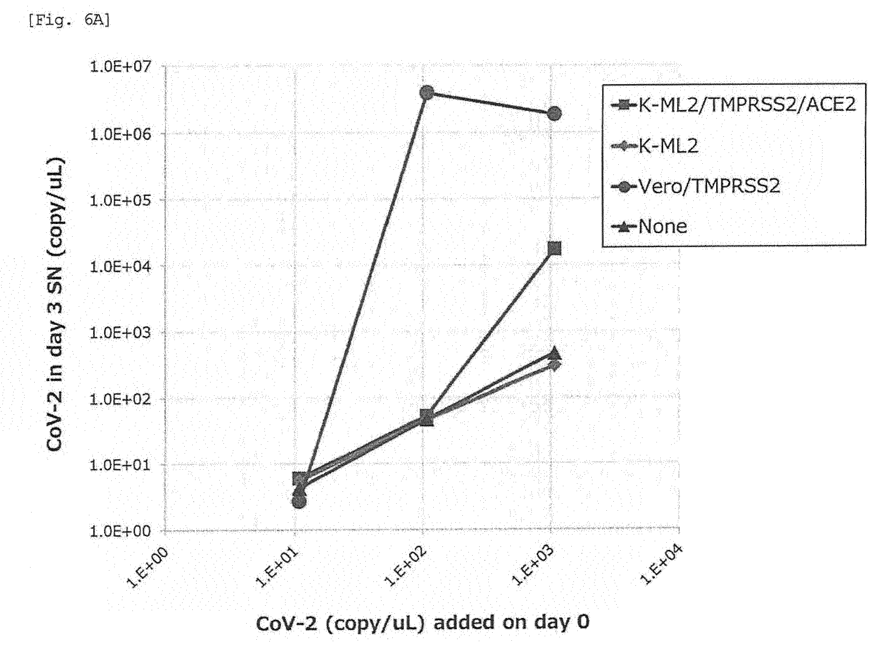

Fig. 6A is a graph showing the viral amount on day 3 after infecting K-ML2 expressing ACE2 and TMPRSS2 (K-ML2/TMPRSS2/ACE2), and M-CSF and GM-CSF-expressing immortalized human myeloid cells derived from K strain (K-ML2) with the SARS-CoV-2 virus in a concentration-dependent manner. As a positive control, TMPRSS2-expressing Vero cells (Vero/TMPRSS2) were used, and as a negative control (None), virus was seeded in a cell-free medium. The horizontal axis shows the virus concentration (copy/uL) of SARS-CoV-2 at the time of virus addition (day 0), and the vertical axis shows SARS-CoV-2 virus concentration (copy/µL) in the culture supernatant measured by quantitative PCR on day 3 of culture.

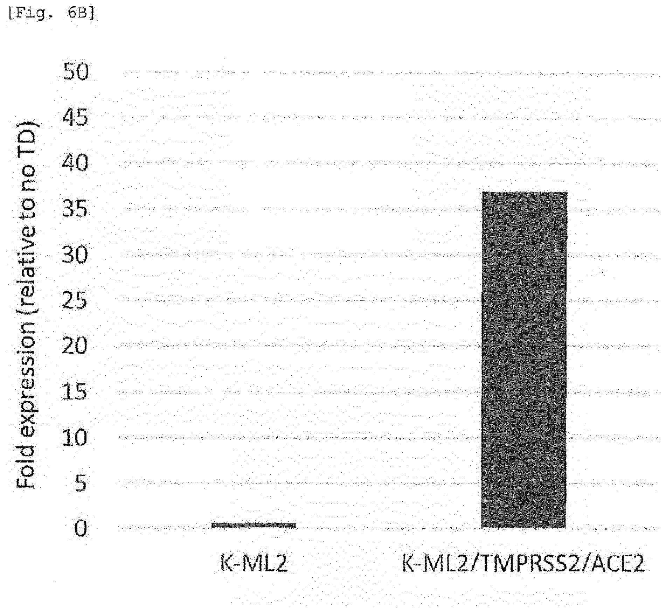

Fig. 6B shows the results of analysis of virus proliferation ability when infected with SARS-CoV-2 virus (1×10³ copy/µL) in Fig. 6A. With the value of the negative control (None) in Fig. 6A as 1, the virus proliferation ability is shown as a proportion of the virus concentration in the culture supernatant of K-ML2 expressing ACE2 and TMPRSS2 (K-ML2/TMPRSS2/ACE2), and M-CSF and GM-CSF-expressing immortalized human myeloid cells derived from K strain (K-ML2) in Fig. 6A. The horizontal axis indicates the sample name, and the vertical axis indicates the value obtained by dividing the virus concentration in the culture supernatant of each cell by the amount of virus in the sample added to a cell-free medium (Fold expression (relative to no TD)).

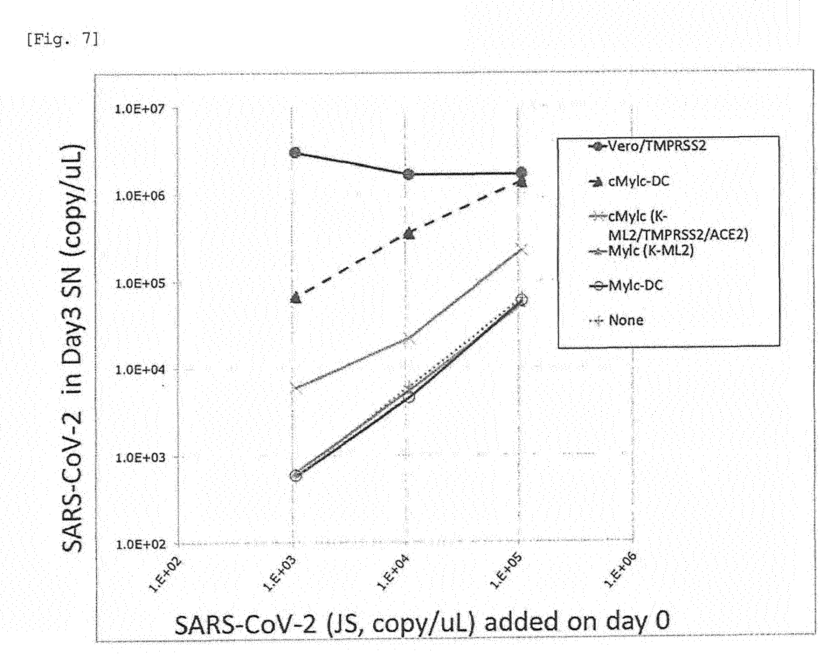

Fig. 7 is a graph showing the viral amount in the culture supernatant on day 3 after infecting M-CSF and GM-CSF-expressing immortalized human myeloid cells (Mylc; K-ML2), dendritic cells obtained by differentiating Mylc (Mylc-DC), Mylc expressing ACE2 and TMPRSS2 (cMylc), and dendritic cells obtained by differentiating cMylc (cMylc-DC) with SARS-CoV-2. As a positive control, TMPRSS2-expressing Vero cells (Vero/TMPRSS2) were used, and as a negative control (None), a cell-free medium was used. The horizontal axis shows the virus concentration (copy/pL) of SARS-CoV-2 at the time of virus addition (day 0), and the vertical axis shows SARS-CoV-2 virus concentration (copy/pL) measured by quantitative PCR 72 hr after culture.

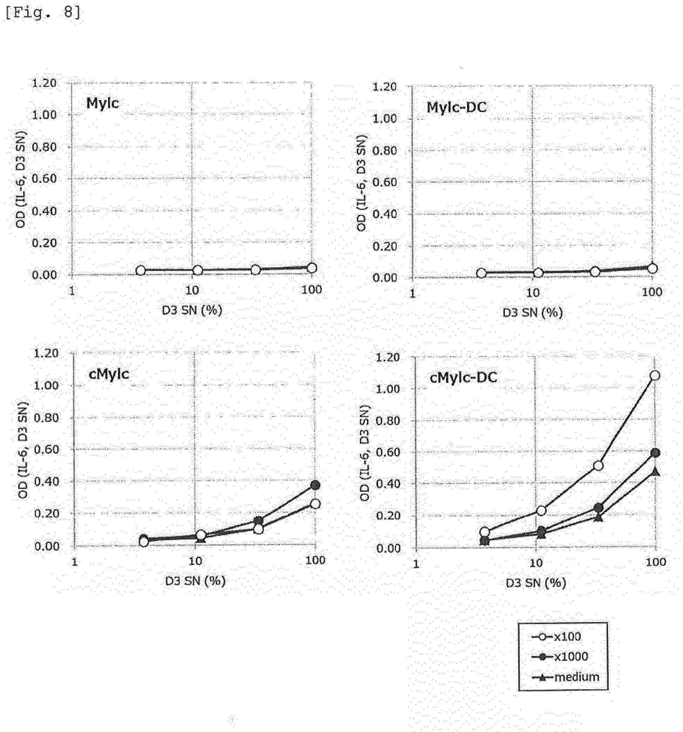

Fig. 8 shows graphs showing the production amounts as measured by ELISA of IL-6 in culture supernatants obtained by culturing Mylc, Mylc-DC, M-CSF and GM-CSF-expressing immortalized human myeloid cells (cMylc), which express ACE2 and TMPRSS2, and dendritic cells obtained by differentiating the same (cMylc-DC), for 72 hr in the presence of SARS-CoV-2. The vertical axis of the graphs shows the measured value of ELISA, and the horizontal axis of the graphs shows the dilution rate of the supernatant cultured for 72 hr. For example, in cMylc-DC, a group in which the supernatant containing the SARS-CoV-2 virus cultured in advance in Vero cells was 100-fold diluted (x100), added to cMylc-DC, cultured for 72 hr, and the supernatant was directly measured by ELISA (horizontal axis 100) indicates about 1.10 on the vertical axis, and a group in which the supernatant was diluted 10-fold before measurement (horizontal axis 10) indicates about 0.20 on the vertical axis, in the line graph with white dots.

### [Description of Embodiments]

### <Definition of terms>

In the present specification, the "myeloid cell" is also called a bone marrow cell and defined as a cell expressing a CD11b molecule or a CD33 molecule, and its origin is not particularly limited. Examples thereof include a myeloid cell derived from a pluripotent stem cell and a myeloid cell collected from a living body (e.g., peripheral blood). Specific examples of the myeloid cell include granulocytes (neutrophils, eosinophils, basophils), monocytes, dendritic cells, macrophages, or more undifferentiated cells destined to differentiate into these cells (myeloid progenitor cells, etc.) in leukocytes.

In the present specification, the "coronavirus" is defined as a single-stranded RNA virus belonging to the Coronaviridae family, which causes respiratory infections such as fever and dry cough. Specifically, SARS-CoV, SARS-CoV-2, Middle East Respiratory Syndrome-associated virus (MARS-CoV), HCoV-229E, HCoV-OC43, HCoV-NL63, HCoV-HKU1, and the like can be mentioned, preferably SARS-CoV-2. In addition, coronaviruses include types that infect not only humans but also livestock, pets, and wild animals. Specific examples of coronaviruses that infect those other than humans include porcine epidemic diarrhea virus (PEDV) and feline infectious peritonitis virus (FIPV). As SARS-CoV-2, for example, "SARS-CoV-2/Hu/DP/Kng/19-020 (GenBank:LC528232)" isolated by the Kanagawa Institute of Public Health can be used.

In the present specification, the "coronavirus-infectious" means being able to be infected by a coronavirus. Thus, a coronavirus-infectious cell means a cell that is subjected to infection with a coronavirus.

In the present specification, the "level" means an index relating to a quantified existing amount, and includes, for example, concentration, amount, and an index that can be used in place thereof. The level may be an absolute numerical value (i.e., measured existing amount) or a relative numerical value compared to a control set as necessary.

### <Preparation method of coronavirus-infectious immortalized human myeloid cells>

In one embodiment, the present invention relates to a method for preparing a coronavirus-infectious immortalized human myeloid cell, including introducing ACE2 and TMPRSS2 into an immortalized human myeloid cell. The immortalized human myeloid cell used in this method may be an immortalized monocyte or a cell prepared by introducing M-CSF and GM-CSF into an immortalized monocyte.

Monocytes can be prepared by a method of collecting from peripheral blood, a method of inducing differentiation from pluripotent stem cells, or a method of inducing differentiation from other somatic cells such as fibroblasts, once put into an undifferentiated state, or by directly inducing differentiation by direct reprogramming.

When monocytes are collected from peripheral blood, the monocytes can be obtained as cells expressing CD14 molecule in human peripheral blood, by known separation and preparation methods. For example, human peripheral blood is gently diluted with an equal amount of saline, phosphate buffered saline, Hanks' buffered solution, or the like, gently overlaid onto Ficoll (registered trademark) (GE Healthcare) in a centrifuge tube, and centrifuged at 15 to 30°C, 500 to 1000×G for 20 min. A white band-like layer between yellowish plasma and transparent Ficoll can be collected as a peripheral blood mononuclear cell (PBMC) fraction consisting of lymphocytes and monocytes. The collected peripheral blood mononuclear cell fraction may be further washed and used if necessary. Monocytes can be obtained by further recovering cells expressing the CD14 molecule from the recovered PBMC fraction. For example, monocytes can be separated and recovered by bringing a solid phase with an anti-CD14 antibody bound thereto and PBMC into contact with each other to allow binding of the monocytes to the solid phase, and removing the unbound cells by washing. For example, a method using magnetic beads as such solid phase and the like are known (Dynabeads (registered trademark) CD14 (Thermo Fisher Scientific Inc.) and the like). Monocytes can also be obtained by directly contacting the peripheral blood with the solid phase to which the anti-CD14 antibody is bound, without separating PBMC from the peripheral blood.

When monocytes are obtained by differentiation from pluripotent cells, a method for inducing monocytes from pluripotent cells is known. In the present specification, the "pluripotent stem cell" means a cell having pluripotency and self-replication ability. In the present specification, the "pluripotency" is synonymous with multipotency and means a state of cell capable of differentiating into cells of multiple lineages by differentiation. Therefore, the "pluripotent stem cell" in the present specification includes stem cell, embryonic stem (ES) cell, embryonic stem cells derived from a clone embryo obtained by nuclear transfer ("ntES cell"), germ stem cell ("GS cell"), embryonic germ cell ("EG cell"), induced pluripotent stem (iPS) cell, and hematopoietic stem cell. As to whether or not a cell is a pluripotent stem cell, for example, when a test cell forms an embryoid body in an ex-vivo culture system, or when it is differentiated into a desired cell after being cultured (differentiation treatment) under differentiation-inducing conditions, the cell can be determined to be a pluripotent stem cell.

Induction of differentiation of pluripotent stem cells into monocytes can be performed by contacting, for example, macrophage colony stimulating factor (M-CSF) and interleukin 3 (IL-3) (Fernadndo O. Martinez et al., Experimental Hematology (2008); 36:1167-1175), or IFN-γ or PMA (Annabelle Grolleau et al., J Immunol 1999; 162:3491-3497), or macrophage colony stimulating factor (M-CSF) and granulocyte macrophage colony stimulating factor (GM-CSF) (WO2012/043651, JP-A-2017-131136, and JP-A-2018-171005) with pluripotent stem cells. The monocytes obtained by inducing differentiation may be purified by recovering only cells expressing CD14 by the aforementioned method as necessary.

Monocytes can be immortalized by introducing at least one gene selected from BMI1 gene, EZH2 gene, MDM2 gene, MDM4 gene, HIF1A gene, BCL2 gene and LYL1 gene, and cMYC gene into the monocytes obtained above, thereby adding proliferation potency thereto, while maintaining the function of monocyte. Preferred is a combined use of at least one gene selected from BMI1 gene, EZH2 gene, MDM2 gene, MDM4 gene, and BCL2 gene, and cMYC gene; or a combination of BMI1 gene and cMYC gene. Introduction of these genes into monocytes can be performed in consideration of the descriptions in WO2012/043651 and JP-A-2017-131136. Introduction of genes can be performed by the gene introduction methods described in the following.

Immortalized monocytes can maintain their proliferation capacity by further introducing M-CSF and GM-CSF. Thus, the method of the present invention may include further introducing M-CSF and GM-CSF into immortalized myeloid cells or immortalized monocytes. Alternatively, cells expressing M-CSF and GM-CSF may be used as the immortalized myeloid cells or immortalized monocyte monocytes. Introduction of these genes into immortalized myeloid cells or immortalized monocyte monocytes can be performed by reference to the description of JP-A-2018-171005.

Coronavirus-infectious immortalized human myeloid cells can be prepared by introducing ACE2 and TMRPSS2 genes into the immortalized human monocytes obtained above, according to the method described in the gene transfer method described below. More specifically, these genes may be introduced by lentiviral vectors.

ACE2 is an abbreviation for angiotensin converting enzyme II, and is an essential counterregulatory carboxypeptidase of the renin-angiotensin hormone system, which is an important regulator of cardiovascular homeostasis. A human ACE2 protein typically has the amino acid sequence described in Table 2 or SEQ ID NO:10.

TMPRSS2 is an abbreviation for type II transmembrane serine protease, and is a plasma membrane-anchored serine protease involved in the proteolytic cascade associated with the normal physiological function of the prostate. The human TMPRSS2 protein typically has the amino acid sequence described in Table 2 or SEQ ID NO:8.

In addition, ACE2 and TMPRSS2 to be introduced into cells may be isoforms and variants thereof, variants in which 1 to 5 or 1 to 3 amino acids have been substituted, inserted, deleted, or added, or variants of ACE2 and TMPRSS2 to be introduced and having 80% or more, 85% or more, 90% or more, 95% or more, 98% or more, or 99% or more, identity with the aforementioned ACE2 and TMPRSS2, as long as the cells into which they have been introduced acquire infectivity or improved infectivity for coronavirus.

For example, since ACE2 is cleaved by ADAM17 and binding to coronavirus is inhibited, ACE2 having a mutation in the cleavage site by ADAM17 may also be used. An example of such mutation is a mutation in which the 706th methionine in the amino acid sequence shown in SEQ ID NO: 10 in Table 2 is substituted with proline. Also, given a report that mutation of the 26th and 27th lysine and threonine in the amino acid sequence enhance the binding of SARS-CoV-2 to ACE2 (doi:https://doi.org/10.1101/2020.04.07.024752), in ACE2, the 26th lysine may be substituted with arginine and/or the 27th threonine may be substituted with proline in SEQ ID NO: 10. In order to increase the binding capacity of ACE2 and SARS-CoV-2, a variant in which the 584th leucine of ACE2 is substituted with alanine can be used (Am J Physiol Lung Cell Mol Physiol. 2009 Jul; 297(1) :L84-96.).

### <Method for introducing gene>

A known method can be used and the method is not particularly limited as long as the desired effect is achieved. Examples of methods for introducing genes include methods for introducing desired.genes into cells by using vectors such as virus, plasmid, artificial chromosome, and the like, or techniques such as lipofection, liposome, microinjection, electroporation, and the like. Examples of the virus vector include retrovirus vector, lentivirus vector (mentioned above, Cell, 126, pp.663-676, 2006; Cell, 131, pp.861-872, 2007; Science, 318, pp.1917-1920, 2007), adenovirus vector (Science, 322, 945-949, 2008), adeno-associated virus vector, Sendai virus vector (WO 2010/008054), and the like. Examples of the artificial chromosome vector include human artificial chromosome (HAC), yeast artificial chromosome (YAC), bacterium artificial chromosome (BAC, PAC), and the like. As the plasmid, a plasmid for mammalian cells can be used (Science, 322:949-953, 2008). A nucleic acid of interest can be integrated into the host cell genome non-virally using a transposon and, for example, a PiggyBac vector and the like can be used.

The vector can contain regulatory sequences such as promoter, enhancer, ribosome-binding sequence, terminator, and polyadenylation site, so that the gene of interest can be expressed. Where necessary, drug resistance genes (e.g., kanamycin resistance gene, ampicillin resistance gene, puromycin resistance gene, neomycin resistance gene, and the like), selectable marker sequences (e.g., thymidine kinase gene, diphtheria toxin gene, and the like), reporter gene sequences (e.g., fluorescent protein genes such as green fluorescent protein, β-galactosidase gene, luciferase gene, etc.), and the like can be contained.

In addition, the vector may have a replication origin that is functional in mammalian cells and a gene encoding a protein that binds to the replication origin and controls replication, so that it will be replicated and exist episomally even without integration into the chromosome. Examples of the replication origin functional in mammalian cells and the protein that binds to the replication origin and controls replication include the replication origin oriP and EBNA-1 gene for EBV and the replication origin ori and the SV40 large T antigen gene for SV40 (WO 2009/115295, WO 2009/157201, and WO 2009/149233). In addition, an expression vector for polycistronic expression may also be used to simultaneously introduce a plurality of desired genes. For polycistronic expression, the sequences encoding the genes may be ligated via Internal Ribosome Entry Site (IRES) or foot-and-mouth disease virus (FMDV) 2A coding region (Science, 322:949-953, 2008 and WO 2009/0920 422009/152529).

Cells into which the gene of interest has been introduced can be obtained as viable cells by culturing the transfected cells in the presence of a drug corresponding to the drug resistance gene incorporated into the cell, when the vector has a drug resistance gene. When the vector has a reporter gene, they can be obtained as cells with luminescence or fluorescence by performing a luminescence or fluorescence assay corresponding to the reporter gene incorporated into the cell. Such luminescence or fluorescence assay may be performed using a commercially available kit. In addition, a cell in which expression of the target gene mRNA has been confirmed may be defined as a cell into which the target gene has been introduced, by performing quantitative PCR using DNA extracted from the cell as a template. Quantitative PCR may also be performed using a commercially available kit. Alternatively, the target protein produced by the cells may be detected by ELISA or the like, and the cells with confirmed protein expression of the target gene may be taken as the cells into which the target gene has been introduced.

### <Coronavirus-infectious immortalized human myeloid cell>

One embodiment of the present invention relates to an immortalized human myeloid cell that expresses ACE2 and TMPRSS2. The immortalized human myeloid cell may be an immortalized human monocyte or human dendritic cell that expresses ACE2 and TMPRSS2. In such immortalized human myeloid cell, M-CSF and/or GM-CSF may be preferably introduced or expressed.

### <Coronavirus-infectious human dendritic cell>

The immortalized monocytes with ACE2 and TMPRSS2 introduced thereinto which are obtained as mentioned above can be differentiated into dendritic cells by inducing differentiation thereof. In the present specification, therefore, the coronavirus-infectious immortalized human myeloid cells include such dendritic cells that express ACE2 and TMPRSS2. Also, the method of the present invention may include differentiating immortalized human monocytes into dendritic cells. Induction of differentiation from monocytes into dendritic cells can be performed by reference to previous reports (Francoise Chapuis et al., Eur J Immunol. (1997) 27(2):431-41.; Marc Dauer et al., J Immunol (2003) 170(8):4069-4076.; Figdor CG et al., Nat Med. (2004) 10(5):475-80; Helmut Jonuleit et al., Eur J Immunol. (1997) 27(12):3135-42). For example, monocytes can be induced to differentiate into dendritic cells by culturing the monocytes in the presence of 10 - 500 ng/ml IL-4.

The aforementioned coronavirus-infectious immortalized human myeloid cells, coronavirus-infectious immortalized human monocytes, and coronavirus-infectious human dendritic cells can be used for coronavirus infection. These cells can also be made into a composition for coronavirus infection with other components.

### <Method for determining the presence of coronavirus>

One embodiment of the present invention relates to a method for determining the presence of a coronavirus in a sample. Specifically, the method includes adding a sample to a culture solution of coronavirus-infectious immortalized human myeloid cells, and detecting coronavirus or measuring the level of coronavirus in the culture supernatant of the monocytes or dendritic cells.

Examples of the medium to be used for the determination in the present invention include IMDM, RPMI1640, α-MEM, MEM, DMEM, and the like. As an additive, human or bovine plasma protein fraction, bovine fetal serum or human serum, saccharide such as glucose, D-mannitol or the like, amino acid, nucleic acid base such as adenine or the like, sodium hydrogen phosphate, A-tocopherol, linoleic acid, cholesterol, sodium selenite, human holotransferrin, human insulin, ethanolamine, 2-mercaptoethanol, G-CSF, GM-CSF, sodium hydrogen carbonate, methylcellulose or the like may be contained. Where necessary, antibiotic such as gentamicin, ampicillin, penicillin, streptomycin or the like; inorganic salt; buffering agent such as HEPES, phosphate buffering agent, acetate buffering agent, or the like may be added. In a specific example when evaluation is performed using immortalized monocytes, a culture medium obtained by adding 10 - 20% bovine fetal serum or 2 - 5% HPL (Human platelet lysate, human platelet-derived supplement (AdvantaCell) to α-MEM can be used. When evaluation is performed using dendritic cells, IL-4 can be used as an additive and, for example, 50 - 200 ng/mL IL-4-containing medium can be used.

The culture condition may include about 37°C, and may include about 5% CO₂.

As the "sample", human or animal-derived body fluids (blood, nasal discharge, sputum, urine, etc.), or tissues (oropharynx, nasopharynx, etc.), cultured cells, cell cultures (cell culture supernatant), drinking water and food, environmental cleaning and wiping fluids, and the like can be used, for example, blood samples can be used. These may be appropriately concentrated or diluted, or subjected to a pretreatment such as extraction or purification. In the present specification, the "blood sample" includes whole blood, plasma, serum, fractionated or processed blood, such as hemolysate of whole blood or blood cells, and diluents and concentrates thereof, preferably serum or diluents thereof. When the sample is a liquid, a diluted solution made at any dilution fold may be used for evaluation.

The aforementioned coronavirus-infectious immortalized human myeloid cells (including coronavirus-infectious immortalized human monocytes and coronavirus-infectious human dendritic cells, hereinafter the same) are seeded and cultured in the aforementioned medium, the cells are passaged or the medium is changed at predetermined times and intervals as necessary, and then a sample can be added. After addition of the sample, the cells are further cultured while exchanging the medium at predetermined times and intervals as necessary. The culture period after the addition of the sample may be of one type, or of two or more types. For example, the culture period may be 1-7 days, 2-6 days, 2-5 days, 2-4 days, or 3 days.

The number of passages or medium change may be, for example, once, twice, three times, four times, or five times or more. The interval of passage or medium exchange may be, for example, once every 2 to 3 days, once every 4 to 5 days, or once a week.

The detection of coronavirus or measurement of the level of coronavirus in the culture supernatant after the completion of culture can be performed by detecting coronavirus RNA or measuring coronavirus RNA levels, plaque assay, virus titer measurement such as TCID50, or the like. After the completion of culture, measurement methods using RNA as an index are often used for rapid and quantitative detection and measurement. The detection of coronavirus RNA or measurement of the RNA level of coronavirus is not particularly limited as long as RNA can be detected or the amount of RNA can be measured. In general, a method utilizing a substance that specifically binds to coronavirus RNA is performed. In one embodiment, the "substance that specifically binds to coronavirus RNA " may be a nucleic acid molecule, preferably a nucleic acid molecule having a sequence complementary to the coronavirus RNA. The nucleic acid molecule having a sequence complementary to the coronavirus RNA can specifically bind to coronavirus RNA by hybridization. In the present specification, the "nucleic acid" includes DNA, RNA, or an artificially created nucleic acid (including locked nucleic acids such as PNA, Locked Nucleic Acid (2',4'-BNA), and the like), and combinations thereof. The nucleic acid molecule that specifically binds to coronavirus RNA may contain an artificially designed sequence (e.g., a sequence for labeling or tagging) in at least a part thereof.

The "probe" is a nucleic acid molecule that typically has a sequence complementary to a coronavirus RNA sequence and is used to measure binding to a coronavirus RNA sequence. The probe is generally a nucleic acid molecule of 10-30 mer, 10-20 mer, etc. that can specifically bind to coronavirus RNA. Examples of a method for measuring the binding level between coronavirus RNA and a probe include Southern hybridization, Northern hybridization, dot hybridization, fluorescence in situ hybridization (FISH), microarray, and ASO method. Specifically, a method using GeneChipTM miRNA Array Strip (Thermo Fisher Scientific) or Agilent miRNA microarray (Agilent Technologies) can be used.

The detection of coronavirus RNA or measurement of the RNA level of coronavirus can be performed by detecting the binding of coronavirus RNA bound to a substance that specifically binds to coronavirus RNA, or measuring the binding level. The "binding level" can be the amount of binding, the number of binding, or the ratio of binding, or a numerical value representing them (e.g., the measured value itself such as the measured fluorescence intensity). In this case, a labeled substance may be used as a substance that specifically binds to coronavirus RNA, or a labeled coronavirus RNA may be used. In general, a standard sample is measured at the same time, a standard curve or calibration curve is created based on the standard sample, and a value calculated from the measured value of the measurement sample, or a numerical value normalized using the standard sample level as an index is determined as the binding level.

The labeling method includes, for example, radioisotope (RI) labeling, fluorescent labeling, and enzymatic labeling.

For example, the coronavirus RNA level may be measured by the following (a) to (c):
(a) contacting a nucleic acid molecule (probe) that binds to at least one coronavirus RNA base sequence or a part thereof with the aforementioned culture supernatant;
(b) measuring the binding level of coronavirus RNA in the aforementioned culture supernatant bound to the aforementioned probe; and
(c) determining the presence of coronavirus in the aforementioned culture supernatant from the measured binding level.

Alternatively, the coronavirus RNA may be detected by the following (a) to (c):
(a) contacting a nucleic acid molecule (probe) that binds to at least one coronavirus RNA base sequence or a part thereof with the aforementioned culture supernatant;
(b) detecting the binding of coronavirus RNA in the aforementioned culture supernatant bound to the aforementioned probe; and
(c) when the binding is detected, determining the presence of coronavirus in the aforementioned culture supernatant.

Alternatively, a method using PCR can be utilized for the detection of coronavirus RNA or measurement of the RNA level of coronavirus. For example, it may be measured by performing qPCR, ARMS (Amplification Refractory Mutation System), RT-PCR (Reverse transcriptase-PCR), or Nested PCR, using a nucleic acid (primer) that specifically binds to coronavirus RNA. Alternatively, Invader assay (registered trademark) may also be used. The "primer" is generally a nucleic acid molecule of 10-30 mer (preferably 17-25 mer, 15-20 mer, etc.), that is used for nucleic acid amplification and has a sequence complementary to the terminal sequence of coronavirus RNA in at least a part thereof (preferably 7 mer or more, 8 mer or more, 9 mer or more, 10 mer or more). As the primers, for example, nucleic acid molecules having the base sequence described in SEQ ID NOs: 11 - 14 or consisting of the base sequence described in SEQ ID NOs: 11 - 14 can be used.

Therefore, the coronavirus RNA level may be measured by the following steps:
(a) amplifying all or part of the coronavirus RNA in the aforementioned culture supernatant by using the aforementioned culture supernatant as a template and using a nucleic acid molecule (primer) that can specifically bind to coronavirus RNA;
(b) measuring the level of amplified nucleic acid molecule; and
(c) determining the presence of coronavirus in the aforementioned culture supernatant from the level of amplified nucleic acid molecule.

Also, the coronavirus RNA may be detected by the following steps:
(a) amplifying all or part of the coronavirus RNA in the aforementioned culture supernatant by using the aforementioned culture supernatant as a template and using a nucleic acid molecule (primer) that can specifically bind to coronavirus RNA;
(b) detecting amplified nucleic acid molecule; and
(c) determining the presence of coronavirus in the aforementioned culture supernatant when amplified nucleic acid molecule is detected.

All or part of the coronavirus RNA in the culture supernatant can be amplified by performing PCR reaction and the like using the culture supernatant as a template. The level of amplified nucleic acid can be measured by dot blot hybridization method, surface plasmon resonance method (SPR method), PCR-RFLP method, in situ RT-PCR method, PCR-SSO (sequence specific oligonucleotide) method, PCR-SSP method, AMPFLP (amplified fragment length polymorphism) method, MVR-PCR method, or PCR-SSCP (single strand conformation polymorphism) method.

In the present specification, "specifically binds" to RNA means that the substance binds to a nucleic acid having coronavirus RNA sequence with affinity substantially higher than the affinity for nucleic acids having other base sequences. Here, the "substantially high affinity" means affinity of a level that makes it possible to detect a nucleic acid having coronavirus RNA sequence as distinguished from nucleic acids having other base sequences. Other base sequences are preferably distinguishably different from coronavirus RNA sequence, and may have 50% or less, 40% or less, 30% or less, 20% or less, or 10% or less identity. For example, substantially high affinity may be an amount of binding to coronavirus RNA of 3 times or more, 4 times or more, 5 times or more, 6 times or more, 7 times or more, 8 times or more, 9 times or more, 10 times or more, 15 times or more, 20 times or more, 30 times or more, 50 times or more, the amount of binding to other base sequence.

Whether a coronavirus is present in a sample is determined based on the detected coronavirus RNA or measured coronavirus RNA level. Specifically, when coronavirus RNA is detected in the aforementioned culture supernatant, it may be determined that coronavirus is present in the sample. When the measured amount of coronavirus RNA level in the culture supernatant increases as compared with the negative control, it may be determined that coronavirus is present in the sample. As the negative control, the medium used in this evaluation may also be used.

Alternatively, the detection of coronavirus or measurement of level of coronavirus in culture supernatant after completion of culture can be performed by detection of coronavirus protein or measurement of the protein level of coronavirus. The detection of coronavirus protein or measurement of the protein level of coronavirus is not particularly limited as long as protein can be detected or the amount of protein can be measured. In general, a method utilizing a substance that specifically binds to coronavirus protein is performed. In one embodiment, as the "substance that specifically binds to coronavirus protein", antibody or antigen-binding fragment thereof, aptamer, ligand/receptor or binding fragment thereof, or fusion product thereof with other substance can be mentioned.

In the present specification, the "antigen binding fragment" means a protein or peptide containing a part (partial fragment) of an antibody and retaining the action (immunoreactivity/bindability) of the antibody to the antigen. Examples of the antigen binding fragment include F(ab')₂, Fab', Fab, Fab₃, single chain Fv (hereinafter to be referred to as "scFv"), (tandem) bispecific single chain Fv (sc(Fv)₂), single chain triple body, nanobody, divalent VHH, pentavalent VHH, minibody, (double-stranded) diabody, tandem diabody, bispecific tribody, bispecific bibody, dual affinity retargeting molecule (DART), triabody (or tribody), tetrabody (or [sc(Fv)₂]₂), or (scFv-SA)₄) disulfide-stabilized Fv (hereinafter to be referred to as "dsFv"), compact IgG, heavy chain antibody, and polymers thereof (Nature Biotechnology, 29(1):5-6 (2011); Maneesh Jain et al., TRENDS in Biotechnology, 25(7) (2007):307-316; and Christoph stein et al., Antibodies(1):88-123 (2012)). In the present specification, the antibody and immunoreactive fragment may be any of monospecific, bispecific (bispecificity), trispecific (trispecificity), and multispecific (multispecificity).

Typically, detection of coronavirus protein or measurement of coronavirus protein level is performed by detecting binding or measuring binding level of a coronavirus protein bound to a substance that specifically binds to the coronavirus protein. The "binding level" to be measured may be the binding amount, binding number, or binding ratio of these substances, or a numerical value showing them (e.g., the measured value itself such as measured fluorescence intensity). In this case, a labeled substance may be used as a substance that specifically binds to a coronavirus protein, or a labeled coronavirus protein may be used. In general, a standard sample is measured at the same time, and a value calculated by forming a standard curve or calibration curve based on the standard sample, or a numerical value normalized using the standard sample level as an index, is determined as the binding level.

Therefore, for example, the method of the present invention may include the following:
(a) contacting a substance that binds to at least one coronavirus protein with the aforementioned culture supernatant;
(b) detecting the binding of or measuring the binding level of a coronavirus protein in the aforementioned culture supernatant bound to a substance that binds to the aforementioned coronavirus protein; and
(c) determining the presence of coronavirus in the aforementioned culture supernatant from the binding detected or the binding level measured.

In the present specification, when a protein is detected or measured using an antibody or an antigen binding fragment thereof, the binding can be measured based on a known detection and/or measurement method. For example, the binding can be measured by labeled immunoassay methods such as enzyme immunoassay (EIA method), simplified EIA method, Enzyme-linked Immunosorbent Assay method (ELISA method), radioimmunoassay (for RIA method), fluorescence immunoassay (FIA method), and the like; immunoblotting methods such as Western blotting method and the like; immunochromatography such as colloidal gold agglutination method and the like; chromatography methods such as ion exchange chromatography method, affinity chromatography method, and the like; turbidimetric method (TIA method); nephelometric method (NIA method); colorimetric method; latex agglutination (LIA method); particle counting method (CIA method); chemiluminescent measurement method (CLIA method, CLEIA method); sedimentation method; surface plasmon resonance method (SPR method); resonant mirror detector method (RMD method); comparative interferometry, and the like.

Specifically, the aforementioned culture supernatant is brought into contact with the antibody of the present invention or an antigen-binding fragment thereof, which is immobilized on a solid phase, after washing, a labeled antibody capable of binding to the protein to be detected or measured is added, unbound antibody is removed by washing, and the label of the antibody is detected or the amount of label (e.g. intensity of label) is measured, whereby the protein to be detected or measured can be detected or the level thereof can be determined. When immunochromatography is performed, the aforementioned culture supernatant is brought into contact with a first labeled antibody capable of binding to the protein to be detected or measured, which antibody is not immobilized, the mixture is brought into contact with a carrier on which a second antibody capable of binding to the protein to be detected or measured or an antigen binding fragment thereof is immobilized at a specific site, and the aforementioned labeled antibody or the amount of label thereof (e.g., intensity of label) at the site is detected or measured, whereby the protein to be detected or measured can be detected or the level thereof can be determined. The labeling method includes, for example, radioisotope (RI) labeling, fluorescent labeling, and enzymatic labeling.

In the present specification, that a protein "specifically binds" means that the substance binds to a nucleic acid having coronavirus protein sequence with affinity substantially higher than the affinity for protein having other amino acid sequences. Here, the "substantially high affinity" means affinity of a level that makes it possible to detect a coronavirus protein as distinguished from proteins having other amino acid sequences. Other amino acid sequences are preferably distinguishably different from coronavirus protein sequence, and may have 50% or less, 40% or less, 30% or less, 20% or less, or 10% or less identity. For example, substantially high affinity may be an amount of binding to coronavirus protein of 3 times or more, 4 times or more, 5 times or more, 6 times or more, 7 times or more, 8 times or more, 9 times or more, 10 times or more, 15 times or more, 20 times or more, 30 times or more, 50 times or more, the amount of binding to other amino acid sequence.

Whether a coronavirus is present in a sample is determined based on the detected coronavirus protein or measured coronavirus protein level. Specifically, when coronavirus protein is detected in the aforementioned culture supernatant, it may be determined that coronavirus is present in the sample. When the measured amount of coronavirus protein level in the culture supernatant increases as compared with the negative control, it may be determined that coronavirus is present in the sample. As the negative control, the medium used in this evaluation may also be used.

Alternatively, the detection of coronavirus or measurement of level of coronavirus in culture supernatant after completion of culture can be performed by detection of inflammatory cytokine or measurement of the inflammatory cytokine level. Generally, inflammatory cytokines produced by immune cells due to viral infection cause inflammation of internal organs and aggravate viral infections. Evaluation of the production amount of such inflammatory cytokine in virus-infected cells is expected to lead to elucidation of the pathology of aggravation and treatment methods. As the inflammatory cytokine, for example, IL-1β, IL-6, IL-8, TNF-α, MIP-1β, and the like can be mentioned.

The detection of inflammatory cytokine or measurement of the level of inflammatory cytokine is not particularly limited as long as protein can be detected or the amount of protein can be measured. In general, a method utilizing a substance that specifically binds to inflammatory cytokine is performed. In one embodiment, as the "substance that specifically binds to inflammatory cytokine", antibody or antigen-binding fragment thereof, aptamer, ligand/receptor or binding fragment thereof, or fusion product thereof with other substance can be mentioned.

Typically, detection of inflammatory cytokine or measurement of inflammatory cytokine level is performed by detecting binding or measuring the binding level of inflammatory cytokine bound to a substance that specifically binds to the inflammatory cytokine. The "binding level" to be measured may be the binding amount, binding number, or binding ratio of these substances, or a numerical value showing them (e.g., the measured value itself such as measured fluorescence intensity). In this case, a labeled substance may be used as a substance that specifically binds to inflammatory cytokine, or a labeled inflammatory cytokine may be used. In general, a standard sample is measured at the same time, and a value calculated by forming a standard curve or calibration curve based on the standard sample, or a numerical value normalized using the standard sample level as an index, is determined as the binding level.

Therefore, for example, the method of the present invention may include the following:
(a) contacting a substance that binds to at least one inflammatory cytokine with the aforementioned culture supernatant;
(b) detecting the binding of or measuring the binding level of inflammatory cytokine in the aforementioned culture supernatant bound to a substance that binds to the aforementioned inflammatory cytokine; and
(c) determining the presence of coronavirus in the aforementioned culture supernatant from the binding detected or the binding level measured.

Whether a coronavirus is present in a sample is determined based on the detected inflammatory cytokine or measured inflammatory cytokine level. Specifically, when inflammatory cytokine is detected in the aforementioned culture supernatant, it may be determined that coronavirus is present in the sample. When the measured amount of inflammatory cytokine level in the culture supernatant is higher than that of the negative control, it may be determined that coronavirus is present in the sample. As the negative control, the medium used in this evaluation may also be used.

### <Production method of coronavirus>

One embodiment of the present invention relates to a production method of coronavirus. Specifically, the production method includes adding coronavirus to a culture solution of coronavirus-infectious immortalized human myeloid cells (including immortalized human monocytes and human dendritic cells) to infect the cells, and recovering the coronavirus in the culture solution of the cells.

Immortalized human myeloid cells are seeded in a medium, the cells are passaged or the medium is changed at predetermined times and intervals as necessary, and then a coronavirus is added to the cultured cells. As the coronavirus to infect the cells, for example, the culture supernatant of a culture solution of cells that has already been infected with coronavirus may be used as it is, or where necessary, it may be diluted with a diluent such as a medium and used. After addition of the sample, the cells are further cultured while exchanging the medium at predetermined times and intervals as necessary. The culture period after the addition of the coronavirus may be 1-7 days, 2-6 days, 2-5 days, 2-4 days, or 3 days. Other culture conditions of immortalized human myeloid cells may follow the conditions described in the aforementioned <Method for determining the presence of coronavirus>.

The culture solution (e.g., culture supernatant) after completion of the culture can be used as it is as a coronavirus-containing solution. Alternatively, the culture solution (e.g., culture supernatant) after completion of the culture can be appropriately concentrated or purified to give a coronavirus-containing solution.

### <Evaluation method of anti-coronavirus activity of test sample>

The present invention relates to a method for evaluating an anti-coronavirus activity of a test sample. The evaluation method includes adding coronavirus to a culture solution of coronavirus-infectious immortalized human myeloid cells (including immortalized human monocytes and human dendritic cells) in the presence of a test sample, and measuring the level of coronavirus in the culture supernatant.

In the present specification, the "anti-coronavirus activity" means an activity that provides therapeutic effects, symptom-relieving effects, and/or preventive effects against coronavirus infections. Typically, it is neutralizing activity or proliferation inhibitory activity (e.g., RNA strand synthesis inhibitory activity, RNA polymerase inhibitory activity, and the like).

Immortalized human myeloid cells are seeded in a medium, the cells are passaged or the medium is changed at predetermined times and intervals as necessary, and then a coronavirus is added to the cultured cells. As the coronavirus to infect the cells, for example, the culture supernatant of a culture solution of cells that has already been infected with coronavirus may be used as it is, or where necessary, it may be diluted with a diluent such as a medium and used. After addition of the sample, the cells are further cultured while exchanging the medium at predetermined times and intervals as necessary. The culture period after the addition of the coronavirus may be 1-7 days, 2-6 days, 2-5 days, 2-4 days, or 3 days. Other culture conditions of immortalized human myeloid cells may follow the conditions described in the aforementioned <Method for determining the presence of coronavirus>.

A test sample can be added to a cell culture solution before addition of coronavirus, simultaneously with coronavirus addition, or after coronavirus addition. For example, in order to confirm neutralization activity and prophylactic effects, it is preferably added before coronavirus addition or simultaneously with coronavirus addition. For example, a method for evaluating an anti-coronavirus activity of a test sample includes
adding a test sample to a culture solution of coronavirus-infectious immortalized human myeloid cells,
adding a coronavirus to the aforementioned cell culture solution, and
measuring the level of the aforementioned coronavirus in a culture supernatant of the aforementioned cells, and
evaluating the test sample as having an anti-coronavirus activity when the level of the coronavirus in the culture supernatant to which the coronavirus has been added in the presence of the test sample is lower than the level of the coronavirus in the culture supernatant to which the coronavirus has been added in the absence of the test sample.

Alternatively, the evaluation method of the anti-coronavirus activity of a test sample of the present invention may be a method for evaluating the effects of a test sample on already infected cells. Specifically, it may include
adding a coronavirus to a culture solution of coronavirus-infectious immortalized human myeloid cells,
adding a test sample to the aforementioned cell culture solution, and
measuring the level of the aforementioned coronavirus in a culture supernatant of the aforementioned cells, and
evaluating the test sample as having an anti-coronavirus activity when the level of the coronavirus in the culture supernatant to which the coronavirus has been added in the presence of the test sample is lower than the level of the coronavirus in the culture supernatant to which the coronavirus has been added in the absence of the test sample.

The modality of the test sample is not limited, and may be, for example, antibody, receptor or a fragment thereof, middle-molecule, peptide, small molecule, aptamer, or nucleic acid drug. A test sample diluted to an any dilution series may be used for evaluation. In addition, the test sample does not need to consist of a single substance, and may be human or animal-derived body fluids (blood, nasal discharge, sputum, urine, etc.), or tissues (oropharynx, nasopharynx, etc.), cultured cells, cell cultures (cell culture supernatant), drinking water and food, environmental cleaning and wiping fluids, and the like can be used, for example, biological components such as blood sample, mixture, composition, plant extract, or the like. For example, by using a blood sample obtained from a subject with a history of coronavirus infection or a subject who has received administration of a coronavirus vaccine or a candidate thereof as a test sample, whether the component (typically an antibody) in the blood of the subject has anti-coronavirus activity can be determined. Alternatively, the test sample may be a test drug consisting only of a single component, wherein solutions and diluents such as solvents or medium components are removed.

The cell culture period after addition of the coronavirus and test sample may be set to any period. For example, the culture period may be 1-7 days, 2-6 days, 2-5 days, 2-4 days, or 3 days. Other culture conditions of immortalized human myeloid cells may follow the conditions described in the aforementioned <Method for determining the presence of coronavirus>. The coronavirus level in the culture supernatant after lapse of the aforementioned culture period can be measured according to the aforementioned <Method for determining the presence of coronavirus>.

Whether the test sample has an anti-coronavirus activity is determined by comparing the measured coronavirus level in the culture supernatant with the coronavirus level of the control. As the control, a culture supernatant of the cells cultured together with the coronavirus in the absence of the test sample can be used. Specifically, the test sample may be determined to have an anti-coronavirus activity when the level of the coronavirus in the culture supernatant containing the test sample is lower than the level of the coronavirus in the culture supernatant free of the test sample.

### identification method of anti-coronavirus substance in test sample>

The present invention relates to a method for identifying whether a substance having an anti-coronavirus activity (anti-coronavirus substance) is contained in a test sample. The identification method of the present invention includes blending a coronavirus to a culture solution of coronavirus-infectious immortalized human myeloid cells (including immortalized human monocytes and human dendritic cells) in the presence of a test sample, measuring the level of the aforementioned coronavirus in a culture supernatant of the aforementioned cells, and identifying that the test sample contains a substance having an anti-coronavirus activity when the level of the coronavirus in the culture supernatant to which the coronavirus has been added in the presence of the test sample is lower than the level of the coronavirus in the culture supernatant to which the coronavirus has been added in the absence of the test sample.

In the method of the present invention, the "test sample" is a sample for which determination is desired as to whether or not it contains an anti-coronavirus substance. Any sample can be used as long as the method of the present invention can be applied. A sample that does not have cytotoxicity by itself is preferred and, for example, the aforementioned blood sample, food, drink, plant extract, and the like can be used. For example, it may be an antibody, or blood or serum or an extract after vaccine inoculation. A test substance diluted to any dilution series may be used for evaluation.

For example, an anti-coronavirus substance to be identified by the identification method of the present invention can follow the aforementioned modality, and may be a substance having coronavirus-neutralizing activity, preferably a coronavirus-neutralizing antibody.

Each specific step in the identification method of anti-coronavirus substance in a test sample can be performed according to the aforementioned <Evaluation method of anti-coronavirus activity of test sample>.

Whether the test sample contains an anti-coronavirus substance is identified by comparing the measured coronavirus level in the culture supernatant with the coronavirus level of the control. As the control, a culture supernatant of the cells cultured together with the coronavirus in the absence of the test sample can be used. Specifically, the test sample may be determined to contain an anti-coronavirus substance when the level of the coronavirus in the culture supernatant containing the test sample is lower than the level of the coronavirus in the culture supernatant free of the test sample.

### <Evaluation method for coronavirus vaccine>

The evaluation method of the anti-coronavirus activity of a test sample of the present invention, and the identification method of an anti-coronavirus substance in a test sample of the present invention can be used to determine whether a neutralizing antibody is produced in vivo by administration of a vaccine (or whether an antibody produced by administration of a vaccine has neutralizing activity). Specifically, blood is collected from mammals (mouse, rat, guinea pig, rabbit, dog, pig, cow, horse, monkey, human, etc.) to which vaccine was administered, and a blood sample prepared from the blood is used as a test sample, and the evaluation method of the anti-coronavirus activity of a test sample of the present invention, or the identification method of an anti-coronavirus substance in a test sample of the present invention is performed. When the test sample is determined to have anti-coronavirus activity or is identified as containing an anti-coronavirus substance, it can be determined that neutralizing antibody is produced in vivo by administration of the vaccine (or antibody produced by administration of the vaccine has neutralizing activity). Therefore, the present invention relates to a method for evaluating a neutralizing antibody inducibility of a coronavirus vaccine, the method including performing the evaluation method of the anti-coronavirus activity of a test sample of the present invention, or the identification method of the coronavirus neutralization substance in a test sample of the present invention, by using a blood sample derived from a mammal inoculated with the vaccine as a test sample.

The evaluation method of coronavirus vaccines of the present invention may further contain a step of administering a vaccine, and may be, for example, an evaluation method including inoculating a vaccine candidate substance to a test mammal, collecting blood from the test mammal, and performing the evaluation method of the anti-coronavirus activity of a test sample of the present invention, or the identification method of the coronavirus neutralization substance in a test sample of the present invention, by using the collected blood or a blood sample prepared from the blood as a test sample.

### <Kit>

The present invention relates to a kit for determining the presence of coronavirus, a kit for producing coronavirus, a kit for evaluating anti-coronavirus activity of a test sample, a kit for identifying an anti-coronavirus substance in a test sample, or a kit for evaluating coronavirus vaccine, each of which contains coronavirus-infectious immortalized human myeloid cells. In addition to the coronavirus-infectious immortalized human myeloid cells, the kit may include a package and instructions. In addition, the kit may include one or more kinds selected from culture medium, culture additives, a substance capable of binding to coronavirus RNA or protein, a standard substance, and the like. As the culture medium, the medium described in the <Method for determining the presence of coronavirus> can be used.

The present invention is explained in more detail in the following based on Examples; however, the present invention is not limited by the following Examples. All documents cited throughout this application are incorporated herein by reference in their entirety.

### (Example 1) (Production of immortalized monocyte cells derived from human induced pluripotent stem cell)

Immortalized monocyte cells derived from human induced pluripotent stem (iPS) cells were produced by reference to previous reports (WO 2012/043651, JP-A-2017-131136 and JP-A-2018-171005). Specifically, undifferentiated iPS cells (K strain ((WO 2012/043651, JP-A-2017-131136 and JP-A-2018-171005)) were inoculated on a 10 cm diameter dish (AGC TECHNO GLASS Co., Ltd., Cat#:1012-100) or 6-well plate (Corning Inc., Cat#: 3516) coated with laminin 511 (iMatrix-511-E8, Nippi, Inc., Cat#: 892-012) as a basement membrane in advance, and differentiation-induction was started using a culture medium obtained by adding 20% bovine fetal serum (hereinafter to be indicated as FBS) (Cytiva Inc., Cat#:SH30088.03) to α-MEM medium (Fuji film Wako Pure Chemical Industries, Ltd., Cat#: 137-17215) (hereinafter to be indicated as α-MEM medium (containing 20% FBS)). Thereafter, the culture was continued while exchanging the α-MEM medium (containing 20% FBS) once every 3 days. At 14 to 21 days after the start of differentiation induction, equal amounts of TrypLE^{™} Select (containing 1 mM ethylenediaminetetraacetic acid (EDTA)) (GIBCO Inc., Cat#: A12859-01) and 0.5 mM EDTA/PBS (Nacalai Tesque INC., Cat#: 13567-84) were mixed, and the cells were dissociated by treating (37°C, 60 min) with the prepared solution (final concentration 0.75 mM EDTA) and collected, and a cell suspension was prepared by pipetting. Subsequently, the cell suspension collected from one dish with a diameter of 10 cm or one well of a 6-well plate was suspended in 10 mL of D-MEM medium (Fujifilm Wako Pure Chemical Industries, Ltd., Cat#: 044-29765) (containing 10% bovine fetal serum (hereinafter to be indicated as FBS) (Cytiva Inc., Cat#: SH30088.03)), and seeded on two dishes or one dish with a diameter of 10 cm and left standing in a 5% CO₂ incubator at 37°C. After 16 hr, the floating cells that did not adhere to the dish (diameter 10 cm) were collected and passed through a 100 µm mesh cell strainer (Corning Inc., Cat#:352360) to obtain a cell suspension after removal of coagulated cell aggregates.

The cell suspension obtained above was mixed with a culture medium containing α-MEM medium (containing 20% FBS, 100 ng/mL M-CSF (Peprotech Inc., Cat#: AF-300-25), and 100 ng/mL GM-CSF (Peprotech Inc., Cat#: 300-03)) and seeded and cultured in a T25 flask (CellSeed Inc., HydroCell, Cat#: CSF025, or Thermo Fisher Scientific Inc., Cat#: 169900). Thereafter, about 3 to 9 days later, floating or weakly adhesive cells appeared, and it was observed that the number of cells gradually increased.

Into the obtained floating cells or weakly-adhesive cells were introduced c-MYC and BMI1 by using a third-generation lentivirus vector (SignaGen Inc.) which is a strain deficient in human immunodeficiency virus type 1 (HIV-I) proliferation potential, etc., and whose promoter for protein expression is EF1a, to produce immortalized monocyte cells derived from human induced pluripotent stem cell. The immortalized monocyte cell line was cultured in α-MEM medium containing 20% FBS, 50-100 ng/mL M-CSF, and 50-100 ng/mL GM-CSF in a 24-well plate in a 5% CO₂ incubator at 37°C, and acquired as proliferative cells 3 to 5 weeks after the start of culture. The immortalized monocyte cells derived from human induced pluripotent stem cells were stored in liquid nitrogen using a commercially available cell storage solution, for example, a serum-free cell storage solution BAMBANKER (GCLTEC Inc., Cat#: CS-02-001).

### (Example 2) Production of immortalized monocyte cells expressing M-CSF and GM-CSF derived from human induced pluripotent stem cell

Immortalized monocyte cells expressing M-CSF and GM-CSF derived from human induced pluripotent stem cell were prepared by reference to a previous report (JP-A- 2018-171005). Specifically, using a third-generation lentivirus vector (SignaGen Inc.) which is a strain deficient in human immunodeficiency virus type 1 (HIV-I) proliferation potential, etc., and whose promoter for protein expression is EF1a, and an expression vector for human M-CSF gene, and an expression vector for human GM-CSF gene were simultaneously introduced into the immortalized monocyte cells derived from human induced pluripotent stem cells obtained in Example 1. From 3 days after gene transfer, the cells were cultured in α-MEM (containing 20% FBS) medium not containing both human M-CSF and GM-CSF in a 5% CO₂ incubator at 37°C, acquired as proliferative cells 3 to 5 weeks after the start of culture, and stored in liquid nitrogen using a commercially available cell storage solution.

### (Example 3) Production by induction of dendritic cells from immortalized monocyte cells expressing M-CSF and GM-CSF derived from human induced pluripotent stem cell

Dendritic cells were prepared from immortalized monocyte cells expressing M-CSF and GM-CSF derived from human induced pluripotent stem cell by reference to a previous report (JP-A-2018-171005). To be specific, the immortalized monocyte cells expressing M-CSF and GM-CSF derived from human induced pluripotent stem cell produced in Example 2 were suspended immediately or after thawing of stored cells in a culture medium obtained by adding 10% bovine fetal serum (hereinafter to be indicated as FBS) (Cytiva Inc., Cat#:SH30088.03) to α-MEM medium (Fuji film Wako Pure Chemical Industries, Ltd., Cat#: 137-17215) (hereinafter to be indicated as α-MEM medium (containing 10% FBS)) and 100 ng/mL IL-4 (Peprotech Inc., Cat#:200-04), and the cells were cultured in T25 flask in a 5% CO₂ incubator at 37°C for 3 days to induce dendritic cells.

In order to confirm that the prepared cells were monocytoid cells and that the induced cells were dendritic cells, the measurement of surface markers, observation of the morphology by Giemsa staining, and the like were performed. The surface markers were measured using a CD14 antibody, a CD54 antibody, and a CD209 antibody (all Biolegend Inc., Cat#:367103, Cat#:353107, Cat#:330107) and a flow cytometer Accuri of BD Bioscience Inc. As for Giemsa staining, a commercially available Giemsa staining solution (Merck Inc., Cat#:HX69072204) was used to perform Giemsa staining or May-Giemsa staining (Nacalai Tesque, May-Grunwald's Solution, Cat#:37126-14) according to the protocol provided by the manufacturer, and the measurement was performed with a high-magnification microscope (Orimpus, IX70, 400-1000x). The measurement results are shown in Fig. 1. In immortalized monocyte cells expressing M-CSF and GM-CSF derived from human induced pluripotent stem cell, expression of CD14 and CD54 was confirmed. When the cells were induced into dendritic cells, attenuation of expression of CD14 and enhancement of expression of CD54 and CD209, which are indicators of changes in general dendritic cell induction, could be confirmed (Fig. 1A). Moreover, from the results of morphological observation, it was confirmed that they were monocytoid cells (Fig. 1B) and dendritic cells.

The M-CSF and GM-CSF-expressing immortalized human monocyte cells produced from K strain are hereinafter to be referred to as "K-ML2".

### (Example 4) Confirmation of gene expression in immortalized human myeloid cells

Many studies have conventionally been conducted on the infection mechanism of coronaviruses to humans. When coronaviruses such as SARS-CoV-2 infect humans, it is important to cause the fusion of the viral outer membrane and the cell membrane. It is known that the spike protein (S protein) in the structure binds to the ACE2 receptor on the cell membrane of human cells, then the S protein is activated by undergoing cleavage by the proteolytic enzyme TMPRSS2, thus achieving the fusion of viral outer membrane and cell membrane (Markus Hoffmann et al., Cell, 2020 April; 181, 271-280).

Therefore, in order to infect K-ML2 with SARS-CoV-2 more efficiently, gene expression of ACE2 that binds to SARS-CoV-2, and TMPRSS2 and ADAM17 that are important for the intracellular invasion of SARS-CoV-2 in immortalized human myeloid cells was analyzed.

Expression of each gene in K-ML2 prepared in Example 2 was detected using the primer set (Eurofins Inc.) shown in Table 1, PCR solution (Takara Bio Inc., One Step TB Green PrimeScript^{™} RT-PCR Kit II, Cat#:RR086A), and real-time PCR equipment (Step One Plus, Applied Biosystems Inc.). The PCR reaction involved reaction at 50°C for 2 min and 95°C for 2 min, followed by three steps of denaturation at 95°C for 20 sec, annealing at 54-56°C for 30 sec, and extension at 72°C for 30 sec, which were performed for 40 cycles.

**[Table 1]**

| | | | |
|---|---|---|---|
| primer name | primer sequence | SEQ ID NO | amplification product |
| hACE2_F | CTCCTTCTCAGCCTTGTTGC | 1 | 372 bp |
| hACE2_R | TCCAGTACTGTAGATGGTGC | 2 | |
| hTMPRSS2_F | CTCTACGGACCAAACTTCATC | 3 | 153 bp |
| hTMPRSS2_R | CCACTATTCCTTGGGTAGAGTAA | 4 | |
| hADAM17_F | GTCGTGGTGGTGGATGGTAAAA | 5 | 148 bp |
| hADAM17_R | GCCCCATCTGTGTTGATTCTGA | 6 | |

As a result, as shown in Fig. 2, expression of ADAM17 was observed. On the other hand, ACE2 showed expression but the expression level thereof was very small, and almost no expression of TMPRSS2 was detected. Therefore, it was considered necessary to stably express TMPRSS2 and ACE2 in order to increase the infection efficiency of SARS-CoV-2 against K-ML2.

### (Example 5) Introduction of TMPRSS2 gene by lentivirus vector

In order to stably express TMPRSS2 in K-ML2, a third-generation lentiviral vector (SignaGen), which is a strain deficient in human immunodeficiency virus type 1 (HIV-I) proliferation potential and the like, has the TMPRSS2 gene (Table 2: SEQ ID NO: 8) at the downstream of the EF1a promoter and has a drug resistance gene at the downstream of the CMV promoter, was used. K-ML2 cells suspended in α-MEM medium (containing 20% FBS) were seeded in a 24-well plate at 1×10⁶ cells/well and 2×10⁶ TU (titer unit) of virus vector solution was added to introduce the vector. The vector-introduced cells were suspended in α-MEM medium, seeded in a 24-well plate to a final cell number of 1×10⁶ cells/well and cultured in a 5% CO₂ incubator at 37°C. After 4 days of culture, puromycin (Sigma-Aldrich Co. LLC., puromycin, Cat#:P883Cat#:P8833) was added to select gene-introduced cells, and the cells were further cultured for 7 days. Cells that survived due to drug resistance were obtained as TMPRSS2-expressing cells (K-ML2/TMPRSS2).

**[Table 2]**

| name | amino acid sequence | SEQ ID NO |
|---|---|---|
| Human TM PRSS2 protein sequence | MALNSGSPPA IGPYYENHGY QPENPYPAQP TVVPTVYEVH PAQYYPSPVP QYAPRVLTQA SNPVVCTQPK SPSGTVCTSK TKKALCITLT LGTFLVGAAL AAGLLWKFMG SKCSNSGIEC DSSGTCINPS NWCDGVSHCP GGEDENRCVR LYGPNFILQV YSSQRKSWHP VCQDDWNENY GRAACRDMGY KNNFYSSQGI VDDSGSTSFM KLNTSAGNVD IYKKLYHSDA CSSKAVVSLR CIACGVNLNS SRQSRIVGGE SALPGAWPWQ VSLHVQNVHV CGGSIITPEW IVTAAHCVEK PLNNPWHWTA FAGILRQSFM FYGAGYQVEK VISHPNYDSK TKNNDIALMK LQKPLTFNDL VKPVCLPNPG MMLQPEQLCW ISGWGATEEK GKTSEVLNAA KVLLIETQRC NSRYVYDNLI TPAMICAGFL QGNVDSCQGD SGGPLVTSKN NIWWLIGDTS WGSGCAKAYR PGVYGNVMVF TDWIYRQMRA DG | 8 |
| Human ACE2 protein sequence | | 10 |

The results of drug selection are shown in Fig. 3. It was confirmed that the cells in the control group, which was not subject to gene transfer, died immediately after the addition of the drug because they lack drug resistance. In the group into which the TMPRSS2 gene was introduced, the cells proliferated and had drug resistance. Thus, it was expected that a lentiviral vector sequence containing the TMPRSS2 gene had been introduced.

In addition, after selection with the drug, the expression of TMPRSS2 in cells into which the TMPRSS2 gene was introduced was confirmed at the mRNA level. The mRNA amount was detected by the absolute quantification method using the TMPRSS2 primer set in the aforementioned Table 1 and a template of known concentration, PCR solution (Takara Bio Inc., One Step TB Green PrimeScript^{™} RT-PCR Kit II, Cat#:RR086A), and real-time PCR equipment (Step One Plus, Applied Biosystems Inc.). The PCR reaction involved reaction at 50°C for 2 min and 95°C for 2 min, followed by three steps of denaturation at 95°C for 20 sec, annealing at 54°C for 30 sec, and extension at 72°C for 30 sec, which were performed for 40 cycles. The results are shown in Fig. 4. Compared to K-ML2, the expression of TMPRSS2 increased in K-ML2 cells with TMPRSS2 introduced thereinto (K-ML2/TMPRSS2), and introduction of TMPRSS2 could be confirmed.

### (Example 6) Introduction of ACE2 gene by lentivirus vector

The ACE2 gene was introduced into TMPRSS2 gene-introduced K-ML2 cells (K-ML2/TMPRSS2) produced in Example 5, by using a third-generation lentiviral vector (SignaGen), which is a strain deficient in human immunodeficiency virus type 1 (HIV-I) proliferation potential and the like, has ACE2 (SEQ ID NO: 10) at the downstream of the EF1a promoter and has a drug resistance gene which is different from the one used for the aforementioned TMPRSS2 introduction at the downstream of the CMV promoter. K-ML2/TMPRSS2 cells suspended in α-MEM medium (containing 20% FBS) were seeded in a 24-well plate at 1×10⁶ cells/well and 2×10⁶ TU of virus vector solution was added to introduce the vector. The vector-introduced cells were suspended in α-MEM medium, seeded in α-MEM medium (containing 20% FBS) in a 24-well plate to a final cell number of 1×10⁶ cells/well and cultured in a 5% CO₂ incubator at 37°C. After 4 days of culture, gene-introduced cells were selected in the presence of neomycin (G418, Enzo Life Sciences, Cat#:ALX-380-013-G001). As a result, the cells proliferated and had drug resistance in the group into which the ACE2 gene was introduced. Thus, it was expected that a lentiviral vector sequence containing the ACE2 gene had been introduced.

The expression of ACE2 in the cells produced by the aforementioned operation was confirmed by measuring mRNA and protein. The mRNA amount was detected by the absolute quantification method using the ACE2 primer set in the aforementioned Table 1 and a template of known concentration, PCR solution (Takara Bio Inc., One Step TB Green PrimeScript^{™} RT-PCR Kit II, Cat#:A25742), and real-time PCR equipment (Step One Plus, Applied Biosystems Inc.). The PCR reaction involved reaction at 50°C for 2 min and 95°C for 2 min, followed by three steps of denaturation at 95°C for 20 sec, annealing at 54-56°C for 30 sec, and extension at 72°C for 30 sec, which were performed for 40 cycles. The results are shown in Fig. 5. Compared with K-ML2, it was confirmed that the expression of ACE2 increased in the cells (K-ML2/TMPRSS2/ACE2) obtained by introducing ACE2 into the K-ML2/TMPRSS2 produced in Example 5 by the aforementioned operation. In combination with the results in Example 5, therefore, it could be confirmed that TMPRSS2 and ACE2 were introduced into K-ML2 in the cells (K-ML2/TMPRSS2/ACE2) prepared by these operations.

In the following, K-ML2 cells (K-ML2/TMPRSS2/ACE2) with ACE2 and TMPRSS2 introduced thereinto are also referred to as "cMylc" derived from the K strain.

### (Example 7) Measurement of coronavirus infectivity of cMylc

Generally, infectious viruses are detected by (1) direct observation using electron microscope, (2) method by antigen-antibody reaction targeting viral proteins, (3) amplification method of viral gene sequences, and (4) detection of virus-infected cells (dead cells). It is known that, in vero cells which are widely known as SARS-CoV-2 infectious cells, the efficiency of SARS-CoV-2 infection can be measured by quantitative PCR specific for the gene sequence derived from the proliferating virus released into the medium. Therefore, the viral load was measured by quantitative PCR.

cMylc derived from K strain and produced in Example 6 was cultured in α-MEM medium (containing 20% FBS) in a 5% CO₂ incubator at 37°C, and maintained and prepared by exchanging the medium once every 3 days. The cMylc derived from K strain was seeded in a 96 well plate at 2.0×10⁴ cells/well.

As SARS-CoV-2, a virus (SARS-CoV-2/Hu/DP/Kng/19-020) proliferated by inoculating cultured cells (Vero cells) with a sample from an infected person in the Kanagawa Institute of Public Health was used. Culture supernatant released into the culture medium of infected Vero cells was prepared with α-MEM medium (containing 20% FBS) to a final concentration of the virus of 1.0×10¹, 1.0×10², 1.0×10³ copy/µL, added to each well, and cultured in a 5% CO₂ incubator at 37°C. Furthermore, the number of viruses in the culture supernatant on day 3 of culture was measured using quantitative PCR. As the positive control, TMPRSS2-expressing Vero cells (Vero/TMPRSS2) (PLoS One.2019; 14(4):e0215822, purchased from National Institutes of Biomedical Innovation, Health and Nutrition JCRB cell bank (cell No.:JCRB1818)) were used, and α-MEM medium (containing 20% FBS) was used as the negative control.

Specifically, SARS-CoV-2 viral RNA was extracted from the culture supernatant of each cell infected with a virus at a final concentration of 1.0×10¹, 1.0×10², 1.0×10³ copy/µL and subjected to real-time PCR. The viral RNA was extracted using QIAamp Viral RNA Mini Kit (QUIAGEN) and the real-time PCR was performed for detection based on a known method and using a primer set (Table 3, SigmaAldrich) based on the SARS-CoV-2/Hu/DP/Kng/19-020 sequence (Ascension# LC528232.1), a PCR solution (One Step TB Green PrimeScript^{™} RT-PCR Kit II, TaKaRa), and real-time PCR equipment (QuantStudio3, Applied Biosystems). The PCR reaction was performed based on the aforementioned SigmaAldrich primer sequence, under the following two-step conditions of 42°C for 5 min and 95°C for 10 sec, followed by denaturation at 95°C for 5 sec and elongation at 60°C for 34 sec for 40 cycles. The viral load was calculated from the standard curve.

**[Table 3]**

| primer name | primer sequence | SEQ ID NO | amplification product |
|---|---|---|---|
| CoV-2-SYBR-1_F | GCCTCTTCTCGTTCCTCATCAC | 11 | 110 bp |
| CoV-2-SYBH-1_R | AGCAGCATCACCGCCATTG | 12 | |
| CoV-2-SYBR-2_F | AGCCTCTTCTCGTTCCTCATCAC | 13 | 101 bo |
| CoV-2-SYBR-2_R | CCGCCATTGCCAGCCATTC | 14 | |

As shown in Fig. 6A, on day 3 of culture, proliferation of SARS-CoV-2 was confirmed by infection with 1.0×10² copy/µL or more in Vero cells, and 1.0×10³ copy/µL or more in cMylc cells. The results of comparison of the proportion of viral load with respect to the viral load of cell no addition group ((viral load in culture supernatant of each experiment group)/(viral load of cell no addition group)) between K-ML2 and cMylc 3 days after addition of 1.0×10³ copy/µL virus are shown in Fig. 6B. It was shown that introduction of ACE2 and TMPRSS2 into K-ML2 (K-ML2/TMPRSS2/ACE2) promotes proliferation of SARS-CoV-2. From the above, it was confirmed that superior proliferation of SARS-CoV-2 in human cells can be achieved in cMylc.

### (Example 8) Preparation of dendritic cells from immortalized monocyte cells

K-ML2 produced in Example 3 (in order to facilitate understanding of infection comparison with cMylc, hereinafter indicated as K strain-derived Mylc, abbreviated as "Mylc"), and cMylc produced in Example 5 were cultured in α-MEM medium (containing 20% FBS) in a 5% CO₂ incubator at 37°C, and maintained and prepared by exchanging the medium once every 3 days. When Mylc and cMylc were used after cryopreserved once, they were incubated in water at 37°C, suspended in α-MEM medium (containing 20% FBS), centrifuged (300×g) and recovered. This operation was performed twice, and the aforementioned culture and medium change were performed. The prepared cMylc was cultured for 3 days in the presence of 100 ng/mL IL-4 (Peprotech, Cat#:200-04) to cause differentiation into dendritic cells, whereby Mylc or cMylc-derived dendritic cells were obtained. In the following, the dendritic cells obtained by differentiation of Mylc is referred to as "Mylc-DC", and the dendritic cells obtained by differentiation of cMylc is referred to as "cMylc-DC".

### (Example 9) Measurement of coronavirus infectivity of cMylc-DC

As SARS-CoV-2, a virus (SARS-CoV-2/Hu/DP/Kng/19-020) proliferated by inoculating cultured cells (Vero cells) with a sample from an infected person in the Kanagawa Institute of Public Health was used. As the virus, the culture supernatant released into the culture medium of infected Vero cells was used. As the evaluation medium, 10% FBS-containing α-MEM (Gibco, Cat#:C1187500BT) was used.

Mylc-DC and cMylc-DC obtained in Example 8 were cultured in IL-4-containing α-MEM medium (containing 20% FBS and 100 ng/ml IL-4) in a T25 flask, and Mylc obtained in Example 3 and cMylc obtained in Example 5 were cultured in IL-4-free α-MEM medium (containing 20% FBS) in a T25 flask in a 5% CO₂ incubator at 37°C for 3 days. Then, all cells were seeded in a 96-well plate at 1×10⁴ cells/well. As the control group, TMPRSS2-expressing Vero cells (Vero/TMPRSS2) (purchased from National Institutes of Biomedical Innovation, Health and Nutrition JCRB cell bank (cell No.:JCRB1818)) were used.

The prepared SARS-CoV-2 was added to infect Mylc, cMylc, Mylc-DC, or cMylc-DC in a 96-well plate and the cells were cultured in an evaluation medium (containing 10% FBS α-MEM) under 37°C, 5% CO₂ conditions. After 72 hr, the culture supernatant was recovered and the infection amount of progeny virus in the supernatant was measured based on the quantitative PCR method. Specifically, SARS viral RNA was extracted from the culture supernatant of each cell infected with a virus at 1.08×10³, 1.08×10⁴, 1.08×10⁵ TU and subjected to real-time PCR. The primer set, PCR solution, real-time PCR equipment, and real-time PCR conditions were the same as those in Example 7.

The amount of virus released by SARS-CoV-2 sensitization is shown in Fig. 7. Mylc and Mylc-DC showed no difference in the amount of virus release as compared with the negative control (none), whereas cMylc and cMylc-DC showed a significantly higher amount of virus release as compared with the negative control (none). In particular, cMylc-DC showed a higher amount of virus release than cMylc. Thus, it was clarified that more differentiated cells are preferably used in the proliferation of SARS-CoV-2.

### (Example 10) Detection of cytokine expression by coronavirus infection

Immortalized monocyte cells or dendritic cells secrete IL-6 as an inflammatory cytokine. Thus, whether the produced cells express the cytokine by corona infection was confirmed.

The cryopreserved Mylc cells or cMylc cells were incubated in water at 37°C, suspended in α-MEM medium (containing 20% FBS), centrifuged (300×g) and recovered. This operation was performed twice, and the cells were seeded in a culture medium obtained by adding 10% FBS (Cytiva Inc., Cat#:SH30088.03) to a 10 ml of α-MEM (Gibco BRL) or a culture medium added with 2 or 5% HPL (Human platelet lysate, human platelet-derived supplement (AdvantaCell, Cat#:HPCHXCGL50) in a T25 flask and cultured for 3 days.

Mylc-DC and cMylc-DC obtained in Example 8 were cultured in IL-4 (100 ng/ml)-containing α-MEM medium (containing 20% FBS) in a T25 flask, and Mylc and cMylc were cultured in IL-4-free α-MEM medium (containing 20% FBS) in a T25 flask in a 5% CO₂ incubator at 37°C for 3 days. Then, cMylc or cMylc-DC was seeded in a 96-well plate at 2×10⁴ cells/well.

The culture supernatant released into the culture medium of infected Vero cells, which was obtained in the same manner as in Example 9, was 100-fold diluted (×100) or 1000-fold diluted (×1000) with α-MEM medium (containing 10% FBS). The resulting SARS-CoV-2 virus-containing solution was added to infect Mylc, Mylc-DC, cMylc, or cMylc-DC in a 96-well plate, and the cells were cultured in an evaluation medium (α-MEM medium (containing 10% FBS)) under 37°C, 5% CO₂ conditions. The obtained culture supernatant was used for the evaluation. After 72 hr, the culture supernatant was recovered and stored at -80°C until measurement. IL-6 contained in the culture supernatant was measured using TELISA MAX^{™} Deluxe Set HUMAN IL-6 kit, Cat#:430504, manufactured by BioLegend and according to the manufacturer's protocol. The negative control used contained the medium.

The IL-6 production amount by SARS-CoV-2 viral sensitization is shown in Fig. 8. IL-6 secreted by cMylc increased depending on the sensitized viral load and was significantly higher than Mylc. In addition, since the amount of IL-6 in cMylc-DC was even higher than that in cMylc, it was clarified that IL-6 production associated with the SARS-CoV-2 virus is high in more differentiated cells.

Therefore, it was shown that cMylc produced by the method described in this Example and the cells obtained by differentiation thereof (cMylc-DC and the like) allow for infection with and proliferation of SARS-CoV-2 virus. cMylc and differentiated cells thereof enable human cells to perform detection experiments equivalent to SARS-CoV-2 virus infection experiments using conventional non-human cells (e.g. Vero cells).

This application is based on a patent application No. 2020-170141 filed in Japan (filing date: October 7, 2020), the contents of which are incorporated in full herein by reference.

## Claims

1. A method for preparing a coronavirus-infectious immortalized human myeloid cell, comprising introducing ACE2 and TMPRSS2 into an immortalized human myeloid cell.

2. The preparation method according to claim 1, wherein the immortalized human myeloid cell is a cell that expresses M-CSF and/or GM-CSF.

3. The preparation method according to claim 1, comprising introducing M-CSF and/or GM-CSF into the immortalized myeloid cell, and
introducing ACE2 and TMPRSS2 into the obtained immortalized myeloid cell that expresses M-CSF and/or GM-CSF.

4. The preparation method according to any one of claims 1 to 3, wherein the ACE2 and the TMPRSS2 are introduced by lentivirus.

5. The preparation method according to any one of claims 1 to 4, wherein the immortalized human myeloid cell is an immortalized human monocyte.

6. A method for preparing a dendritic cell that expresses ACE2 and TMPRSS2, comprising differentiating the human immortalized monocyte obtained by the method according to claim 5 into a dendritic cell.

7. The preparation method according to any one of claims 1 to 6, wherein the coronavirus is SARS-CoV-2.

8. An immortalized human myeloid cell that expresses ACE2 and TMPRSS2.

9. The immortalized human myeloid cell according to claim 8 that further expresses M-CSF and/or GM-CSF.

10. The cell according to claim 8 or 9, wherein the immortalized human myeloid cell is an immortalized human monocyte.

11. The cell according to claim 8 or 9, wherein the immortalized human myeloid cell is a human dendritic cell.

12. A method for determining the presence of a coronavirus in a sample, comprising adding a sample to a culture solution of the immortalized human myeloid cell according to any one of claims 9 to 11, and
detecting a coronavirus or measuring the level of a coronavirus in a culture supernatant of the cell.

13. The method according to claim 12, wherein the detection of coronavirus or the measurement of the level of coronavirus in the culture supernatant is performed 1 to 7 days after the addition of the sample.

14. The method according to claim 12 or 13, wherein the detection of coronavirus or measurement of the level of coronavirus is performed by detection of RNA or measurement of RNA level of the coronavirus.

15. The method according to claim 14, wherein the detection of RNA or measurement of RNA level of coronavirus is performed by quantitative PCR.

16. The method according to claim 12 or 13, wherein the detection of coronavirus or measurement of the level of coronavirus is performed by detection of an inflammatory cytokine or measurement of the level of inflammatory cytokine in the culture supernatant.

17. The method according to claim 16, wherein the inflammatory cytokine is IL-6.

18. A method for producing a coronavirus, comprising adding a coronavirus to a culture solution of the immortalized human myeloid cell according to any one of claims 9 to 11 to infect the cell, and
recovering the coronavirus in the culture solution of the cell.

19. The method according to claim 18, wherein the coronavirus in the culture solution is recovered 1 to 7 days after the addition of the coronavirus.

20. A method for evaluating an anti-coronavirus activity of a test sample, comprising adding a coronavirus to a culture solution of the immortalized human myeloid cell according to any one of claims 9 to 11 in the presence of the test sample,
measuring the level of the coronavirus in a culture supernatant of the cell, and
evaluating the test sample as having an anti-coronavirus activity when the level of the coronavirus in the culture supernatant to which the coronavirus has been added in the presence of the test sample is lower than the level of the coronavirus in the culture supernatant to which the coronavirus has been added in the absence of the test sample.

21. A method for identifying whether a substance having a coronavirus neutralization action is contained in a test sample, comprising mixing a coronavirus with a culture solution of the immortalized human myeloid cell according to any one of claims 9 to 11 in the presence of the test sample,
measuring the level of the coronavirus in a culture supernatant of the cell, and
evaluating the test sample as containing a substance having a coronavirus neutralization action when the level of the coronavirus in the culture supernatant to which the coronavirus has been added in the presence of the test sample is lower than the level of the coronavirus in the culture supernatant to which the coronavirus has been added in the absence of the test sample.

22. The method according to claim 21, wherein the test sample is a blood sample.

23. The method according to claim 21 or 22, wherein the substance having a coronavirus neutralization action is a coronavirus neutralizing antibody.

24. A method for evaluating a neutralizing antibody inducibility of a coronavirus vaccine, comprising performing the method according to claim 22 or 23 by using a blood sample derived from a mammal inoculated with the vaccine as a test sample.

25. The method according to any one of claims 20 to 24, wherein the measurement of the level of the coronavirus in the culture supernatant is performed 1 to 7 days after the addition of the test sample or the sample.

26. The method according to any one of claims 20 to 25, wherein the detection of coronavirus or measurement of the level of coronavirus is performed by detection of RNA or measurement of RNA level of the coronavirus.

27. The method according to claim 26, wherein the measurement of the RNA level of coronavirus is performed by quantitative PCR.

28. The method according to any one of claims 20 to 25, wherein the measurement of the level of coronavirus is performed by measurement of the level of an inflammatory cytokine in the culture supernatant.

29. The method according to claim 28, wherein the inflammatory cytokine is IL-6.
